# EUROPÄISCHE PATENTANMELDUNG

(11) **EP 2 211 371 A2**
(43) Veröffentlichungstag der Anmeldung: **28.07.2010**
(21) Anmeldenummer: 09015872.6
(22) Anmeldetag: 22.12.2009
(51) Int. Cl.: H01J 61/30, H01J 61/72, A61L 9/015, A61L 9/20

(54) **Gasentladungslampe, Vorrichtung zur Reinigung eines sauerstoffhaltigen Gases und raumlufttechnische Anlage**

(30) Priorität: 23.12.2008 DE 202008017007 U
(71) Anmelder: uv-technik Speziallampen GmbH, 98704 Wümbach (DE)
(72) Erfinder: Meyer, Karl-Heinz, 98704 Wümbach (DE)
(74) Vertreter: Grünecker, Kinkeldey, Stockmair & Schwanhäusser Anwaltssozietät

(57) **Zusammenfassung**

Eine Gasentladungslampe umfasst ein Entladungsgefäß, das mit einem Gas gefüllt ist und zwei Elektroden, die zur Erzeugung einer Gasentladung in dem Entladungsgefäß geeignet sind. Das Gas ist dafür ausgelegt, bei der Gasentladung ozonbildende ultraviolette Strahlung und nicht ozonbildende ultraviolette Strahlung auszusenden. Das Entladungsgefäß weist einen ersten Abschnitt und einen zweiten Abschnitt auf. Der erste Abschnitt ist für die ozonbildende ultraviolette Strahlung durchlässig. Der zweite Abschnitt ist für die ozonbildende ultraviolette Strahlung undurchlässig und für die nicht ozonbildende ultraviolette Strahlung durchlässig. Die Gasentladungslampe kann in einer Vorrichtung zur Reinigung eines sauerstoffhaltigen Gases und/oder in einer raumlufttechnischen Anlage verwendet werden.

## Beschreibung

Die vorliegende Erfindung bezieht sich auf eine Gasentladungslampe, eine Vorrichtung zur Reinigung eines sauerstoffhaltigen Gases, insbesondere zur Reinigung von Luft, und auf eine raumlufttechnische Anlage.

In modernen raumlufttechnischen Anlagen kann neben einer Klimatisierung der Luft auch eine Bestrahlung der Luft mit ultravioletten Strahlen durchgeführt werden, um die Luft zu reinigen. Zu diesem Zweck umfasst die raumlufttechnische Anlage eine ultraviolette Strahlungsquelle.

In raumlufttechnischen Anlagen nach dem Stand der Technik kann die ultraviolette Strahlungsquelle eine Niederdruck-Quecksilberdampflampe umfassen. Diese weist ein Entladungsgefäß, das beispielsweise eine Röhrenform hat, auf. Das Entladungsgefäß ist mit Quecksilberdampf und wahlweise auch einem Edelgas gefüllt. In dem Entladungsgefäß sind zwei Elektroden vorgesehen. Durch Anlegen einer elektrischen Spannung zwischen den Elektroden kann in dem Entladungsgefäß eine elektrische Entladung erzeugt werden, die Quecksilberatome zur Lichtemission anregt.

Quecksilber hat zwei wichtige Spektrallinien im ultravioletten Bereich. Diese haben Wellenlängen von ungefähr 185 nm bzw. ungefähr 254 nm. Ultraviolette Strahlung mit einer Wellenlänge von 254 nm ist besonders wirksam zur Inaktivierung von Mikroorganismen. Ultraviolette Strahlung mit einer Wellenlänge von 185 nm kann ebenfalls Mikroorganismen inaktivieren, und außerdem zur Bildung von Ozon aus dem Luftsauerstoff führen. Bei einer Wellenlänge von 254 nm tritt dagegen keine Bildung von Ozon auf.

Niederdruck-Quecksilberdampflampen nach dem Stand der Technik sind entweder als ozonbildende Lampen oder als ozonfreie Lampen ausgebildet. Bei ozonbildenden Lampen ist das Entladungsgefäß sowohl für Strahlung mit einer Wellenlänge von 185 nm als auch für Strahlung mit einer Wellenlänge von 254 nm durchlässig. Wird Luft oder ein anderes sauerstoffhaltiges Gas mit der Strahlung solcher Lampen bestrahlt, wird Ozon gebildet. Bei ozonfreien Lampen ist das Entladungsgefäß für Strahlung mit einer Wellenlänge von 254 nm durchlässig aber für Strahlung mit einer Wellenlänge von 185 nm undurchlässig, so dass keine Bildung von Ozon auftritt.

Wird in einer raumlufttechnischen Anlage eine ozonfreie ultraviolette Strahlungsquelle verwendet, können Mikroorganismen durch die Strahlung inaktiviert werden. Ein Nachteil, der bei der Verwendung einer ozonfreien ultravioletten Strahlungsquelle auftritt, ist, dass durch eine derartige Bestrahlung keine Geruchsstoffe beseitigt werden können, und dass die Bestrahlung eine relativ geringe Wirkung gegen Mikroorganismen hat, die an Festkörper (z.B. Staubteilchen) gebunden oder von einem Flüssigkeitsfilm überzogen sind.

Wenn in einer raumlufttechnischen Anlage eine ozonbildende ultraviolette Strahlungsquelle verwendet wird, können Mikroorganismen aufgrund der stark oxidierenden Wirkung des Ozons auch dann inaktiviert werden, wenn sie an Festkörper gebunden oder von einem Flüssigkeitsfilm überzogen sind. Außerdem kann das Ozon chemisch mit störenden Geruchsstoffen reagieren und diese in geruchlose Substanzen umwandeln. Ein Nachteil der Verwendung einer ozonbildenden ultravioletten Strahlungsquelle ist, dass gesundheitsschädliche Mengen von Ozon in die Raumluft gelangen können.

Es ist eine Aufgabe der vorliegenden Erfindung, eine Gasentladungslampe, eine Vorrichtung zur Reinigung eines sauerstoffhaltigen Gases und eine raumlufttechnische Anlage bereitzustellen, mit denen die oben genannten Nachteile vermieden werden können.

Eine Gasentladungslampe gemäß der vorliegenden Erfindung umfasst ein Entladungsgefäß, das mit einem Gas gefüllt ist und zwei Elektroden, die zur Erzeugung einer Gasentladung in dem Entladungsgefäß geeignet sind. Das Gas ist dafür ausgelegt, bei der Gasentladung ozonbildende ultraviolette Strahlung und nicht ozonbildende ultraviolette Strahlung auszusenden. Das Entladungsgefäß weist einen ersten Abschnitt, der für die ozonbildende ultraviolette Strahlung durchlässig ist, und einen zweiten Abschnitt, der für die ozonbildende ultraviolette Strahlung undurchlässig und für die nicht ozonbildende ultraviolette Strahlung durchlässig ist, auf.

Wird ein sauerstoffhaltiges Gas, beispielsweise Luft, mit ultraviolettem Licht, das aus dem ersten Abschnitt des Entladungsgefäßes austritt, bestrahlt, kann aus dem Sauerstoff Ozon entstehen. Das Ozon kann, wie oben ausgeführt, Mikroorganismen inaktivieren, selbst wenn diese an Festkörper gebunden oder von einem Flüssigkeitsfilm bedeckt sind. Außerdem können Geruchsstoffe in geruchlose Substanzen umgewandelt werden.

Wird bereits vorhandenes Ozon mit nicht ozonbildender ultraviolette Strahlung, die eine Wellenlänge hat, die nicht dafür geeignet ist, die Bildung von Ozon aus Sauerstoff anzuregen, beispielsweise eine Wellenlänge größer als ungefähr 235 nm bestrahlt, kann dies die Umwandlung des Ozons in zweiatomigen Sauerstoff fördern. Die nicht ozonbildende ultraviolette Strahlung hat somit eine ozonabbauende Wirkung.

Wenn das sauerstoffhaltige Gas nach der Bestrahlung mit ultravioletter Strahlung aus dem ersten Abschnitt des Entladungsgefäßes mit ultravioletter Strahlung aus dem zweiten Abschnitt des Entladungsgefäßes bestrahlt wird, kann deshalb das zunächst erzeugte Ozon wieder abgebaut und in unschädlichen zweiatomigen Sauerstoff umgewandelt werden. Außerdem kann die Strahlung aus dem zweiten Abschnitt des Entladungsgefäßes Mikroorganismen, die nicht an Festkörper gebunden oder von einem Flüssigkeitsfilm bedeckt sind, inaktivieren.

Die erfindungsgemäße Gasentladungslampe kann somit einerseits verwendet werden, um Ozon zu erzeugen, andererseits, um das Ozon wieder abzubauen, wenn es nicht mehr benötigt wird.

Die Gasentladungslampe kann beispielsweise zur Reinigung von Luft in raumlufttechnischen Anlagen verwendet werden. Dabei kann die Luft zuerst an dem ersten Abschnitt des Entladungsgefäßes vorbeigeleitet werden, um Ozon zu erzeugen und anschließend an dem zweiten Abschnitt des Entladungsgefäßes vorbeigeleitet werden, um das Ozon wieder zu zersetzen. Dadurch kann ein unerwünschter Austritt von Ozon in die Raumluft vermieden oder zumindest verringert werden.

In manchen Ausführungsformen kann der erste Abschnitt des Entladungsgefäßes sowohl für die ozonbildende ultraviolette Strahlung als auch für die nicht ozonbildende ultraviolette Strahlung durchlässig sein. Dadurch kann außer der ozonbildenden ultravioletten Strahlung auch die nicht ozonbildende Strahlung aus dem ersten Abschnitt des Entladungsgefäßes austreten und zur Inaktivierung von Mikroorganismen genutzt werden.

In manchen Ausführungsformen kann das Gas Quecksilber umfassen. In solchen Ausführungsformen kann die ozonbildende ultraviolette Strahlung eine Strahlung mit einer Wellenlänge von ungefähr 185 nm umfassen und die nicht ozonbildende Strahlung kann eine Strahlung mit einer Wellenlänge von ungefähr 254 nm umfassen. Diese Wellenlängen entsprechen den Wellenlängen der beiden stärksten ultravioletten Spektrallinien des Quecksilbers. Strahlung mit diesen Wellenlängen kann durch eine Gasentladung in einem quecksilberhaltigen Gas mit gutem Wirkungsgrad erzeugt werden, wodurch ein energiesparender Betrieb der Gasentladungslampe ermöglicht wird.

In manchen Ausführungsformen kann der erste Abschnitt des Entladungsgefäßes aus undotiertem Quarz gebildet sein und der zweite Abschnitt des Entladungsgefäßes kann aus dotiertem Quarz gebildet sein. Beispielsweise kann der dotierte Quarz mit Titan oder Cer dotiert sein. Der undotierte Quarz kann natürlichen und/oder synthetischen Quarz umfassen.

Undotierter natürlicher oder synthetischer Quarz weist für ultraviolette Strahlung eine hohe Durchlässigkeit auf. Durch die Dotierung kann die Durchlässigkeit des Quarzes beeinflusst werden. Insbesondere kann die Durchlässigkeit für Wellenlängen unterhalb einer Grenzwellenlänge, die in manchen Ausführungsformen ungefähr gleich 200 nm sein kann, stark verringert werden. Dadurch können die gewünschten Durchlässigkeitseigenschaften des Entladungsgefäßes im ersten und im zweiten Abschnitt bereitgestellt werden.

In manchen Ausführungsformen umfasst das Entladungsgefäß eine an den Enden geschlossene Röhre. An jedem Ende der Röhre ist eine der Elektroden vorgesehen. Der erste Abschnitt des Entladungsgefäßes befindet sich neben einem ersten der Enden der Röhre und der zweite Abschnitt des Entladungsgefäßes befindet sich neben einem zweiten der Enden der Röhre. Durch diese Ausgestaltung kann man eine aufeinanderfolgende Bestrahlung eines Gases mit ozonbildender und nicht ozonbildender ultravioletter Strahlung besonders einfach erreichen, indem man das Gas in der Richtung vom ersten Ende zum zweiten Ende an der Röhre entlang strömen lässt.

Der erste und der zweite Abschnitt des Entladungsgefäßes können unmittelbar und/oder unlösbar miteinander verbunden sein. Dadurch kann eine mechanisch stabile, dauerhaft beständige und gasdichte Verbindung zwischen den beiden Abschnitten geschaffen werden.

Eine Länge des ersten Abschnitts des Entladungsgefäßes kann kleiner als ein Viertel der Summe der Längen des ersten und des zweiten Abschnitts sein. Dadurch kann sichergestellt werden, dass ein großer Teil des Ozons, das erzeugt wird, wenn ein sauerstoffhaltiges Gas am ersten Abschnitt vorbeiströmt, wieder abgebaut wird, wenn das Gas (mit im Wesentlichen gleicher Strömungsgeschwindigkeit) am zweiten Abschnitt vorbeiströmt.

Eine erfindungsgemäße Vorrichtung zur Reinigung eines sauerstoffhaltigen Gases, insbesondere zur Reinigung von Luft, umfasst eine Gasentladungslampe mit einigen oder allen der oben beschriebenen Merkmale. Außerdem weist die Vorrichtung eine Reinigungskammer auf, in der sich die Gasentladungslampe befindet, sowie Mittel zum Durchströmen der Reinigungskammer mit dem sauerstoffhaltigen Gas. Die Reinigungskammer, die Gasentladungslampe und die Mittel zum Durchströmen der Reinigungskammer mit dem sauerstoffhaltigen Gas sind derart angeordnet, dass das sauerstoffhaltige Gas beim Durchströmen der Reinigungskammer zuerst von der ozonbildenden ultravioletten Strahlung, die aus dem ersten Abschnitt des Entladungsgefäßes austritt, und anschließend von der ultravioletten Strahlung, die aus dem zweiten Abschnitt des Entladungsgefäßes austritt, bestrahlt wird.

Mit Hilfe der Vorrichtung kann zunächst aus dem Sauerstoff Ozon gebildet werden, während das Gas von der ultravioletten Strahlung aus dem ersten Abschnitt der Gasentladungslampe bestrahlt wird. Anschließend kann das Ozon durch die Bestrahlung mir der ultravioletten Strahlung aus dem zweiten Abschnitt wieder abgebaut werden. Dadurch kann das Ozon zur Inaktivierung bzw. Beseitigung von Mikroorganismen und/oder Gerüchen eingesetzt werden. Nachteilige Wirkungen des Ozons wie beispielsweise, im Fall der Reinigung von Luft, gesundheitliche Beeinträchtigungen, können durch den anschließenden Abbau des Ozons vermieden werden.

In manchen Ausführungsformen kann die Gasentladungslampe, wie oben beschrieben, ein röhrenförmiges Entladungsgefäß aufweisen. In solchen Ausführungsformen können die Reinigungskammer, die Gasentladungslampe und die Mittel zum Durchströmen der Reinigungskammer mit dem sauerstoffhaltigen Gas derart angeordnet sein, dass das sauerstoffhaltige Gas beim Durchströmen der Reinigungskammer in einer Richtung vom ersten Ende der Röhre zum zweiten Ende der Röhre an der Röhre entlang strömt.

In manchen Ausführungsformen können die Mittel zum Durchströmen der Reinigungskammer einen Ventilator umfassen.

Eine erfindungsgemäße raumlufttechnische Anlage umfasst eine Vorrichtung zur Reinigung eines sauerstoffhaltigen Gases mit einigen oder allen der oben beschriebenen Merkmale. Mit Hilfe der Vorrichtung können Mikroorganismen und/oder Geruchsstoffe aus der Raumluft entfernt oder inaktiviert werden. Durch den Ozonabbau in der Vorrichtung kann die Ozonkonzentration in der Raumluft auf ein nicht gesundheitsschädliches Maß reduziert werden.

Weitere Ausführungsformen der Erfindung werden mit Bezug auf die beigefügten Figuren beschrieben. Es zeigen:
Fig. 1 eine schematische Zeichnung einer Gasentladungslampe gemäß einer Ausführungsform der vorliegenden Erfindung; und
Fig. 2 eine schematische Querschnittsansicht einer Vorrichtung zur Reinigung eines sauerstoffhaltigen Gases gemäß einer Ausführungsform der vorliegenden Erfindung.

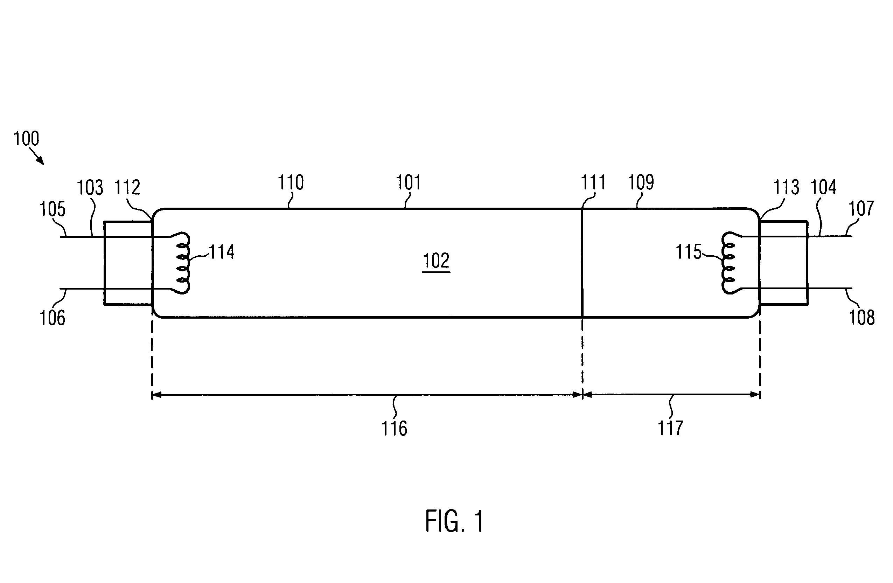

Fig. 1 zeigt eine schematische Zeichnung einer Gasentladungslampe 100 gemäß einer Ausführungsform der vorliegenden Erfindung. Die Gasentladungslampe 100 umfasst ein Entladungsgefäß 101, das mit einem Gas 102 gefüllt ist.

Die Gasentladungslampe 100 kann eine Niederdruck-Quecksilberdampflampe sein. In solchen Ausführungsformen kann das Gas 102 Quecksilber enthalten. Zusätzlich kann das Gas 102 ein Edelgas, beispielsweise Argon, enthalten. Das Gas kann einen Druck von 10 mbar oder weniger aufweisen.

Die Gasentladungslampe 100 umfasst ferner eine erste Elektrode 104 und eine zweite Elektrode 103, die dafür ausgelegt sind, beim Anlegen einer elektrischen Spannung zwischen den Elektroden 103, 104 eine Gasentladung in dem Entladungsgefäß 101 zu erzeugen.

Die Elektroden 103, 104 können Heißkathoden sein. In solchen Ausführungsformen kann die erste Elektrode 104 zwei Anschlüsse 107, 108 sowie einen Heizdraht 115, der zwischen den Anschlüssen 107, 108 verbunden ist, umfassen. Der Heizdraht 115 kann eine Beschichtung aufweisen, welche die Austrittsarbeit von Elektronen aus dem Heizdraht 115 verringert. Die zweite Elektrode 103 kann im wesentlichen genauso aufgebaut sein wie die erste Elektrode 104, wobei die Bezugszeichen 105, 106 die Anschlüsse der Elektrode 103 bezeichnen und das Bezugszeichen 114 den Heizdraht. Beim Starten der Gasentladung können die Heizdrähte 114, 115 durch Anlegen einer elektrischen Spannung zwischen den Anschlüssen 105, 106 und zwischen den Anschlüssen 107, 108 beheizt werden, um eine Zündspannung der Gasentladungslampe 100 zu verringern.

In anderen Ausführungsformen können die Elektroden 103, 104 auch Kaltkathoden sein.

Das Entladungsgefäß 101 kann eine Röhrenform haben. Die Röhre ist an ihren Enden 112, 113 gasdicht verschlossen. Die erste Elektrode 104 ist dabei an einem ersten Ende 113 des Entladungsgefäßes 102 vorgesehen und die zweite Elektrode 103 an einem zweiten Ende 112 des Entladungsgefäßes. Beim Betrieb der Gasentladungslampe 100 kann sich dann die Gasentladung längs durch das Entladungsgefäß 101 erstrecken.

Das Entladungsgefäß 101 weist einen ersten Abschnitt 109 und einen zweiten Abschnitt 110 auf.

Der erste Abschnitt 109 ist für ozonbildende ultraviolette Strahlung durchlässig, die dafür geeignet ist, bei Bestrahlung eines sauerstoffhaltigen Gases wie beispielweise Luft eine chemische Reaktion zwischen zweiatomigen Sauerstoffmolekülen auszulösen, bei der Ozon entsteht. Die ozonbildende ultraviolette Strahlung kann eine Wellenlänge von weniger als 235 nm aufweisen. Zusätzlich kann der erste Abschnitt 109 auch für ultraviolette Strahlung mit größerer Wellenlänge, die bei Bestrahlung eines sauerstoffhaltigen Gases keine Bildung von Ozon auslöst (in Folgenden als "nicht ozonbildende ultraviolette Strahlung" bezeichnet) durchlässig sein.

Der erste Abschnitt 109 des Gasentladungsgefäßes 101 kann in manchen Ausführungsformen aus natürlichem oder synthetischem Quarz gebildet sein, der eine hohe Durchlässigkeit für ozonbildende ultraviolette Strahlung aufweist. Außerdem weist natürlicher oder synthetischer Quarz eine hohe Durchlässigkeit für nicht ozonbildende ultraviolette Strahlen auf.

Der zweite Abschnitt 110 ist für ozonbildende ultraviolette Strahlung undurchlässig, aber durchlässig für nicht ozonbildende ultraviolette Strahlung. Beispielsweise kann der zweite Abschnitt 110 für ultraviolette Strahlung mit einer Wellenlänge kleiner als 200 nm undurchlässig und für ultraviolette Strahlung mit einer Wellenlänge größer als 200 nm durchlässig sein.

In manchen Ausführungsformen kann der zweite Abschnitt 110 des Entladungsgefäßes 101 aus einem dotierten Quarzglas bestehen. Das Quarzglas kann beispielsweise mit Titan oder Cer dotiert sein.

In Ausführungsformen, in denen das Gas 102 Quecksilber enthält, kann das Gas 102 bei der Gasentladung unter anderem ultraviolette Strahlung mit einer Wellenlänge von ungefähr 185 nm und ultraviolette Strahlung mit einer Wellenlänge von ungefähr 254 nm emittieren. Diese beiden Wellenlängen entsprechen den beiden intensivsten Spektrallinien des Quecksilbers im ultravioletten Bereich. Die 185 nm-Strahlung ist ozonbildend, die 254 nm-Strahlung ist nicht ozonbildend. In solchen Ausführungsformen kann der erste Abschnitt 109 des Entladungsgefäßes 101 sowohl für die 185 nm-Strahlung als auch für die 254 nm-Strahlung durchlässig sein und der Abschnitt 110 kann nur für die 254 nm-Strahlung durchlässig sein.

Wird die Gasentladungslampe 100 in einem sauerstoffhaltigen Gas wie beispielsweise Luft betrieben, kann in der Umgebung des ersten Abschnitts 109 Ozon entstehen. In der Umgebung des zweiten Abschnitts 110 wird dagegen nur sehr wenig Ozon oder im Wesentlichen gar kein Ozon gebildet. Die nicht ozonbildende ultraviolette Strahlung kann außerdem zum Abbau von Ozon führen, das in die Umgebung des zweiten Abschnitts 110 strömt, beispielsweise durch eine Gasströmung entlang der Gasentladungsröhre 110 in der Richtung vom ersten Ende 113 zum zweiten Ende 112.

In manchen Ausführungsformen kann das Entladungsgefäß 101 aus zwei Teilen zusammengesetzt sein.

In solchen Ausführungsformen können zunächst ein erster Teil der Gasentladungslampe 100 und ein zweiter Teil der Gasentladungslampe 100 bereitgestellt werden, wobei der erste Teil die erste Elektrode 104 und den ersten Abschnitt 109 des Entladungsgefäßes 101 umfasst und der zweite Teil die zweite Elektrode 103 und den zweiten Abschnitt 110 des Entladungsgefäßes. Der erste und der zweite Teil der Gasentladungslampe können mit bekannten Techniken zur Herstellung von Gasentladungslampen hergestellt werden wobei, wie oben beschrieben, der erste Abschnitt 109 und der zweite Abschnitt 110 des Entladungsgefäßes 101 aus unterschiedlichen Materialien gebildet werden können.

Anschließend werden der erste Abschnitt 109 und der zweite Abschnitt 110 des Entladungsgefäßes 101 miteinander verbunden.

In manchen Ausführungsformen können der erste Abschnitt 109 und der zweite Abschnitt 110 unmittelbar miteinander verbunden werden, beispielsweise durch Verschmelzen des ersten Abschnitts 109 und des zweiten Abschnitts 110 an einer Nahtstelle 111. Zu diesem Zweck können die von den Elektroden 103, 104 abgewandten Enden der Abschnitte 109, 110 erhitzt und zusammengepresst werden. Dies kann entweder manuell oder automatisch mit Hilfe einer Glasdrehbank geschehen. Durch das Verschmelzen des ersten Abschnitts 109 und des zweiten Abschnitts 110 kann eine unlösbare Verbindung zwischen den Abschnitten 109, 110 geschaffen werden.

Nach dem Verbinden der Abschnitte 109, 110 kann das Entladungsgefäß durch einen Befüllungsstutzen (nicht gezeigt) mit dem Gas 102 gefüllt werden, und der Befüllungsstutzen kann zugeschmolzen werden, um das Entladungsgefäß 101 gasdicht zu verschließen.

Der erste Abschnitt 109 und der zweite Abschnitt 110 müssen nicht unmittelbar miteinander verbunden werden. In anderen Ausführungsformen kann ein Zwischenstück (nicht gezeigt) zwischen dem ersten Abschnitt 109 und dem zweiten Abschnitt 110 eingesetzt werden.

In Fig. 1 bezeichnet das Bezugszeichen 117 eine Länge des ersten Abschnitts 109 des Entladungsgefäßes 101, die vom ersten Ende 113 des Entladungsgefäßes 101 bis zur Nahtstelle 111 zwischen den Abschnitten 109, 110 gemessen werden kann. Das Bezugszeichen 116 bezeichnet eine Länge des zweiten Abschnitts 110. Diese kann vom zweiten Ende 114 des Entladungsgefäßes 101 bis zur Nahtstelle 111 gemessen werden.

Die Länge 117 des ersten Abschnitts 109 kann kleiner sein als ein Drittel, insbesondere kleiner als ein Viertel einer Summe der Längen 117, 116 des ersten Abschnitts 109 und des zweiten Abschnitts 110, die im Wesentlichen gleich einer Gesamtlänge des Entladungsgefäßes 101 sein kann. Dadurch kann sichergestellt werden, dass bei im Wesentlichen konstanter Strömungsgeschwindigkeit entlang des Entladungsgefäßes 101 ein großer Teil des in der Umgebung des ersten Abschnitts 109 erzeugten Ozons in der Umgebung des zweiten Abschnitts 110 wieder abgebaut wird. Beispielweise kann sich die Länge 117 in einem Bereich zwischen 5% und 30% der Summe der Längen 116, 117 befinden.

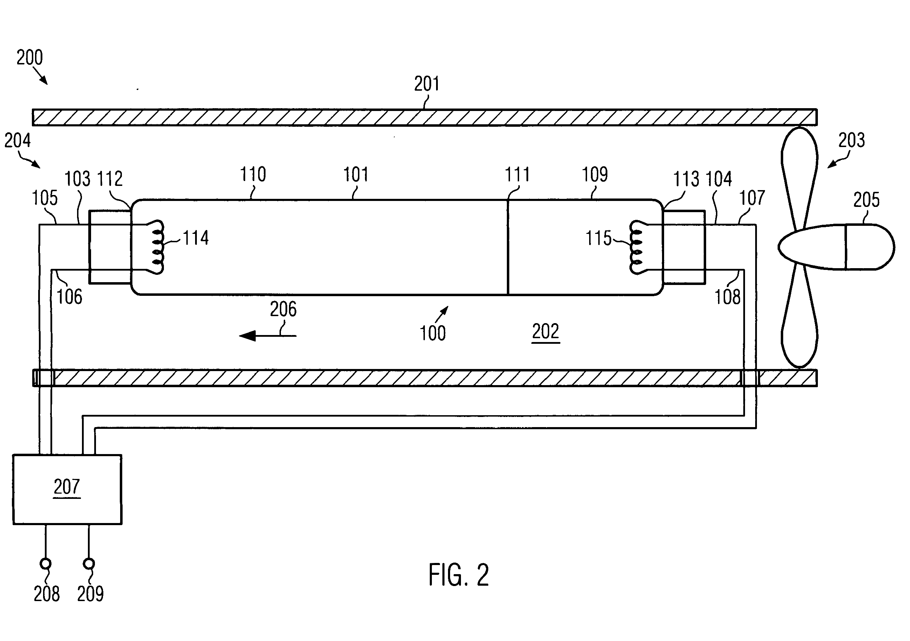

Fig. 2 zeigt eine schematische Querschnittsansicht einer Vorrichtung 200 zur Reinigung eines sauerstoffhaltigen Gases, insbesondere zur Reinigung von Luft. Die Reinigung kann dabei eine Inaktivierung von Mikroorganismen und/oder eine Neutralisierung von Gerüchen umfassen.

Die Vorrichtung 200 umfasst eine Gasentladungslampe 100 mit den oben mit Bezug auf Fig. 1 beschriebenen Merkmalen. Die Anschlüsse 105, 106, 107, 108 der Gasentladungslampe 100 können mit einer Vorschaltelektronik 207 verbunden sein, die durch Anschlüsse 208, 209 mit einer Stromquelle, zum Beispiel mit der Netzspannung, verbunden werden kann. Die Vorschaltelektronik 207 kann dabei der Vorschaltelektronik einer Gasentladungslampe nach dem Stand der Technik entsprechen.

Die Vorrichtung 200 umfasst ferner eine Reinigungskammer 201. Diese weist an zwei einander gegenüberliegenden Enden 203, 204 jeweils eine Öffnung auf, durch die ein sauerstoffhaltiges Gas 202, insbesondere Luft, in die Reinigungskammer 201 eintreten und aus der Reinigungskammer 201 austreten kann. Außerdem sind Mittel zum Durchströmen der Reinigungskammer 201 mit dem Gas 202 vorgesehen, die beispielsweise einen Ventilator 205 umfassen können.

Die Reinigungskammer 201, die Gasentladungslampe 100 und die Mittel zum Durchströmen der Reinigungskammer 201 sind derart angeordnet, dass das Gas 202 zuerst von der ozonbildenden ultravioletten Strahlung, die aus dem ersten Abschnitt 109 des Entladungsgefäßes 101 austritt, und anschließend von der nicht ozonbildenden ultravioletten Strahlung, die aus dem zweiten Abschnitt 110 des Entladungsgefäßes 101 austritt, bestrahlt wird.

Zu diesem Zweck kann die Gasentladungslampe 100 entlang einer Längsrichtung der Reinigungskammer 201 ausgerichtet sein, wobei das erste Ende 113 des Entladungsgefäßes 101 dem ersten Ende 203 der Reinigungskammer 201 zugewandt ist und das zweite Ende 112 des Entladungsgefäßes 101 dem zweiten Ende 204 der Reinigungskammer 201 zugewandt ist.

Der Ventilator 205 kann derart ausgebildet sein, dass die Reinigungskammer 201 in einer Richtung vom ersten Ende 203 zum zweiten Ende 204 von dem Gas 202 durchströmt wird. In Fig. 2 ist die Strömungsrichtung des Gases 202 durch einen Pfeil 206 angedeutet. Dadurch strömt das Gas 202 beim Durchströmen der Reinigungskammer 201 in einer Richtung vom ersten Ende 113 zum zweiten Ende 112 der Gasentladungslampe 100, so dass es zunächst der aus dem ersten Abschnitt 109 austretenden ultravioletten Strahlung und danach der aus dem zweiten Abschnitt 110 austretenden ultravioletten Strahlung ausgesetzt ist.

In manchen Ausführungsformen könnten sich in der Reinigungskammer 201 auch mehrere zueinander im Wesentlichen parallel angeordnete Gasentladungslampen 100 befinden, deren erste Enden 113 dem ersten Ende 203 der Reinigungskammer 201 zugewandt sind.

Die Vorrichtung 200 kann Teil einer raumlufttechnischen Anlage sein, die außer der Vorrichtung 200 beispielsweise Einrichtungen zum Verändern einer Temperatur und/oder einer Luftfeuchtigkeit umfassen kann.

## Patentansprüche

1. Gasentladungslampe (100), umfassend:
ein Entladungsgefäß (101), das mit einem Gas (102) gefüllt ist; und
zwei Elektroden (103, 104), die zur Erzeugung einer Gasentladung in dem Entladungsgefäß (101) geeignet sind;
wobei das Gas (102) dafür ausgelegt ist, bei der Gasentladung ozonbildende ultraviolette Strahlung und nicht ozonbildende ultraviolette Strahlung auszusenden;
wobei das Entladungsgefäß (101) einen ersten Abschnitt (109), der für die ozonbildende ultraviolette Strahlung durchlässig ist, und einen zweiten Abschnitt (110) , der für die ozonbildende ultraviolette Strahlung undurchlässig und für die nicht ozonbildende ultraviolette Strahlung durchlässig ist, aufweist.

2. Gasentladungslampe (100) nach Anspruch 1, wobei der erste Abschnitt (109) des Entladungsgefäßes (101) sowohl für die ozonbildende ultraviolette Strahlung als auch für die nicht ozonbildende ultraviolette Strahlung durchlässig ist.

3. Gasentladungslampe (100) nach einem der vorhergehenden Ansprüche, wobei das Gas (102) Quecksilber umfasst, die ozonbildende ultraviolette Strahlung eine Strahlung mit einer Wellenlänge von 185 nm umfasst und die nicht ozonbildende ultraviolette Strahlung eine Strahlung mit einer Wellenlänge von 254 nm umfasst.

4. Gasentladungslampe (100) nach einem der vorhergehenden Ansprüche, wobei der erste Abschnitt (109) des Entladungsgefäßes (101) aus undotiertem Quarz gebildet ist und der zweite Abschnitt (110) des Entladungsgefäßes (101) aus dotiertem Quarz gebildet ist.

5. Gasentladungslampe (100) nach Anspruch 4, wobei der dotierte Quarz mit Titan und/oder Cer dotiert ist.

6. Gasentladungslampe (100) nach Anspruch 4 oder 5, wobei der undotierte Quarz natürlichen Quarz und/oder synthetischen Quarz umfasst.

7. Gasentladungslampe (100) nach einem der vorhergehenden Ansprüche, wobei das Entladungsgefäß (101) eine an den Enden (112, 113) geschlossene Röhre umfasst, an jedem Ende (112, 113) der Röhre eine der Elektroden (103, 104) vorgesehen ist, sich der erste Abschnitt (109) des Entladungsgefäßes (101) neben einem ersten (113) der Enden der Röhre befindet und sich der zweite Abschnitt (110) des Entladungsgefäßes (101) neben einem zweiten (112) der Enden der Röhre befindet.

8. Gasentladungslampe (100) nach Anspruch 7, wobei der erste (109) und der zweite (110) Abschnitt des Entladungsgefäßes (101) unmittelbar miteinander verbunden sind.

9. Gasentladungslampe (100) nach Anspruch 7 oder 8, wobei der erste (109) und der zweite (110) Abschnitt unlösbar miteinander verbunden sind.

10. Gasentladungslampe (100) nach einem der Ansprüche 7 bis 9, wobei eine Länge (117) des ersten Abschnitts (109) des Entladungsgefäßes (101) kleiner als ein Drittel und/oder kleiner als ein Viertel der Summe der Längen (117, 116) des ersten (109) und des zweiten (110) Abschnitts ist.

11. Vorrichtung (200) zur Reinigung eines sauerstoffhaltigen Gases (202), insbesondere Luft, umfassend:
eine Gasentladungslampe (100) nach einem der Ansprüche 1 bis 10;
eine Reinigungskammer (201) , wobei sich die Gasentladungslampe (100) in der Reinigungskammer (201) befindet;
Mittel zum Durchströmen der Reinigungskammer mit dem sauerstoffhaltigen Gas (202);
wobei die Reinigungskammer (201) , die Gasentladungslampe und die Mittel zum Durchströmen der Reinigungskammer (201) mit dem sauerstoffhaltigen Gas (202) derart angeordnet sind, dass das sauerstoffhaltige Gas (202) beim Durchströmen der Reinigungskammer (201) zuerst von der ozonbildenden ultravioletten Strahlung, die aus dem ersten Abschnitt (109) des Entladungsgefäßes (101) austritt, und anschließend von der nicht ozonbildenden ultravioletten Strahlung, die aus dem zweiten Abschnitt (110) des Entladungsgefäßes (101) austritt, bestrahlt wird.

12. Vorrichtung (200) nach Anspruch 11, wobei die Gasentladungslampe (100) eine Gasentladungslampe nach einem der Ansprüche 7 bis 10 ist, und die Reinigungskammer (201), die Gasentladungslampe (100) und die Mittel zum Durchströmen der Reinigungskammer mit dem sauerstoffhaltigen Gas derart angeordnet sind, dass das sauerstoffhaltige Gas (202) beim Durchströmen der Reinigungskammer (201) in einer Richtung vom ersten Ende (113) der Röhre zum zweiten Ende (112) der Röhre an der Röhre entlang strömt.

13. Vorrichtung (200) nach einem der Ansprüche 11 und 12, wobei die Mittel zum Durchströmen der Reinigungskammer einen Ventilator (205) umfassen.

14. Raumlufttechnische Anlage, umfassend eine Vorrichtung (200) nach einem der Ansprüche 11 bis 13.
